# EUROPEAN PATENT SPECIFICATION

(11) **EP 2 440 514 B1**
(45) Date of publication and mention of the grant of the patent: **22.08.2018**
(21) Application number: 10728495.2
(22) Date of filing: 11.06.2010
(51) Int. Cl.: C07C 209/00, C07C 253/00, C07D 223/10, C08G 63/16, C08G 69/26, C07C 51/377, C07C 55/14, C07C 51/41, C07C 211/12, C07C 255/04, C07D 309/30, C07D 201/08, C07C 51/235, C08G 69/14, C08G 69/36

(54) **PRODUCTION OF ADIPIC ACID AND DERIVATIVES FROM CARBOHYDRATE-CONTAINING MATERIALS**
HERSTELLUNG VON ADIPINSÄURE UND DERIVATEN AUS KOHLENHYDRATHALTIGEN MATERIALIEN
PRODUCTION D'ACIDE ADIPIQUE ET DE DÉRIVÉS DE CELUI-CI À PARTIR DE MATIÈRES CONTENANT DES GLUCIDES

(30) Priority: 13.06.2009 US 268414 P; 05.03.2010 US 311190 P
(43) Date of publication of application: 18.04.2012
(60) Divisional application: 18183021.7
(73) Proprietor: Archer-Daniels-Midland Company, Decatur, IL 62526 (US)
(72) Inventor: BOUSSIE, Thomas, R., Santa Clara, CA 95051 (US); DIAS, Eric, L., Santa Clara, CA 95051 (US); FRESCO, Zachary, M., Santa Clara, CA 95051 (US); MURPHY, Vincent, J., Santa Clara, CA 95051 (US); SHOEMAKER, James, Santa Clara, CA 95051 (US); ARCHER, Raymond, Santa Clara, CA 95051 (US); JIANG, Hong, Santa Clara, CA 95051 (US)
(74) Representative: Potter Clarkson LLP
(86) International application number: PCT/US2010/038408
(87) International publication number: WO 2010/144862

(56) References cited:
- US-A- 3 607 922
- US-A- 5 772 013
- H. DE LA MOTTE: "Ueber die Einwirkung von Phosphorpentachlorid und Jodwasserstoffsäure auf Zuckersäure", BERICHTE DER DEUTSCHEN CHEMISCHEN GESELLSCHAFT, vol. 12, no. 2, 1879, pages 1571-1573, XP002598617, ISSN: 0002-7864, DOI: 10.1002/cber.18790120296
- BESSON, M.; FLECHE, G.; FUERTES, P.; GALLEZOT, P.; LAHMER, F.: "Oxidation of glucose and gluconate on Pt, Pt Bi, and Pt Au catalysts", RECUEIL DES TRAVAUX CHIMIQUES DES PAYS-BAS, vol. 115, no. 4, 1996, pages 217-221, XP008131910, ISSN: 0165-0513, DOI: 10.1002/recl.19961150405
- ABBADI, A.; VAN BEKKUM, H.: "Effect of pH in the Pt-catalyzed oxidation of D-glucose to D-gluconic acid", JOURNAL OF MOLECULAR CATALYSIS A: CHEMICAL, vol. 97, 1995, pages 111-118, XP002617660, ISSN: 1381-1169, DOI: 10.1016/1381-1169(94)00078-6

## Description

### FIELD OF THE INVENTION

The present invention generally relates to processes for the chemocatalytic conversion of a glucose source to an adipic acid product. The present invention includes processes for the conversion of glucose to an adipic acid product via glucaric acid or derivatives thereof. The present invention also includes processes comprising the catalytic oxidation of glucose to glucaric acid and catalytic hydrodeoxygenation of glucaric acid or derivatives thereof to an adipic acid product. The present invention also relates to processes for the preparation of industrial chemicals such as adiponitrile, hexamethylene diamine, caprolactam, caprolactone, 1,6-hexanediol, adipate esters, polyamides (e.g., nylons) and polyesters from an adipic acid product obtained from processes which include the catalytic hydrodeoxygenation of glucaric acid or derivatives thereof.

### BACKGROUND OF THE INVENTION

Crude oil is currently the source of most commodity and specialty organic chemicals. Many of these chemicals are employed in the manufacture of polymers and other materials. Examples include ethylene, propylene, styrene, bisphenol A, terephthalic acid, adipic acid, caprolactam, hexamethylene diamine, adiponitrile, caprolactone, acrylic acid, acrylonitrile, 1,6-hexanediol, 1,3-propanediol, and others. Crude oil is first refined into hydrocarbon intermediates such as ethylene, propylene, benzene, and cyclohexane. These hydrocarbon intermediates are then typically selectively oxidized using various processes to produce the desired chemical. For example, crude oil is refined into cyclohexane which is then selectively oxidized to "KA oil" which is then further oxidized for the production of adipic acid, an important industrial monomer used for the production of nylon 6,6. Many known processes are employed industrially to produce these petrochemicals from precursors found in crude oil. For example, see Ullmann's Encyclopedia of Industrial Chemistry, Wiley 2009 (7th edition).

For many years there has been an interest in using biorenewable materials as a feedstock to replace or supplement crude oil. See, for example, Klass, Biomass for Renewable Energy, Fuels, and Chemicals, Academic Press, 1998. Moreover, there have been efforts to produce adipic acid from renewable resources using processes involving a combination of biocatalytic and chemocatalytic processes. See, for example, "Benzene-Free Synthesis of Adipic Acid", Frost et al. Biotechnol. Prog. 2002, Vol. 18, pp. 201-211, and U.S. Patent Nos. 4,400,468, and 5,487,987. In "Berichte der Deutschen Chemischen Gesellschaft, vol. 12, no.2, 1879, pages 1571-1573, H. de la Motte describes the preparation of adipic acid from saccharic acid using red phosphor.

One of the major challenges for converting biorenewable resources such as carbohydrates (e.g. glucose derived from starch, cellulose or sucrose) to current commodity and specialty chemicals is the selective removal of oxygen atoms from the carbohydrate. Approaches are known for converting carbon-oxygen single bonds to carbon-hydrogen bonds. See, for example: U.S. Patent No. 5,516,960; U.S. Patent App. Pub. US2007/0215484 and Japanese Patent No. 78,144,506. Each of these known approaches suffers from various limitations and we believe that, currently, none of such methods are used industrially for the manufacture of specialty or industrial chemicals.

Thus, there remains a need for new, industrially scalable methods for the selective and commercially-meaningful conversion of carbon-oxygen single bonds to carbon-hydrogen bonds, especially as applied in connection with the production of chemicals from polyhydroxyl-containing substrates (e.g., glucaric acid), and especially for the production of chemicals from polyhydroxyl-containing biorenewable materials (e.g., glucose derived from starch, cellulose or sucrose) to important chemical intermediates such as adipic acid.

### SUMMARY OF THE INVENTION

Briefly, therefore, the present invention is directed to processes for preparing an adipic acid product from polyhydroxyl-containing biorenewable materials. In accordance with one embodiment, a process for producing an adipic acid product from a glucose source is provided which comprises converting by chemocatalytic means at least a portion of the glucose source to the adipic acid product.

In accordance with another embodiment, the process for preparing an adipic acid product comprises reacting, in the presence of a hydrodeoxygenation catalyst and a halogen source, a hydrodeoxygenation substrate and hydrogen to convert at least a portion of the hydrodeoxygenation substrate to an adipic acid product, wherein the hydrodeoxygenation substrate comprises a compound of formula I 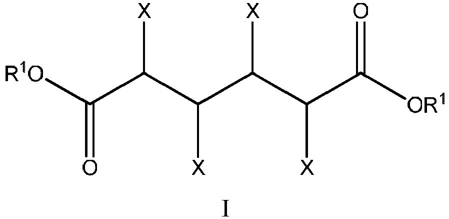 wherein X is independently hydroxyl, oxo, halo, acyloxy or hydrogen provided that at least one X is not hydrogen and R¹ is independently a salt-forming ion, hydrogen, or hydrocarbyl; or a mono- or di-lactone thereof.

In accordance with another embodiment, the process for preparing an adipic acid product comprises converting at least a portion of a glucose source to a hydrodeoxygenation substrate comprising glucaric acid or derivative thereof, and converting at least a portion of the glucaric acid or derivative to an adipic acid product.

The present invention is further directed to processes for preparing glucaric acid. In one embodiment, the process comprises reacting glucose with a source of oxygen in the presence of an oxidation catalyst and in the substantial absence of added base.

The present invention is further directed to processes for preparing glucaric acid by reacting glucose with oxygen in the presence of an oxidation catalyst, wherein at least a portion of the glucose is solubilized with a weak carboxylic acid, preferably acetic acid.

The present invention is further directed to processes for the preparation of industrial chemicals such as adiponitrile, hexamethylene diamine, caprolactam, caprolactone, 1,6-hexanediol, adipate esters, polyamides (e.g., nylons) and polyesters from an adipic acid product obtained from processes for the chemocatalytic conversion of a glucose source, which may include, for example, the catalytic hydrodeoxygenation of glucaric acid or derivatives thereof.

The present invention is further directed to adipic acid product, polyamides, polyesters and caprolactam produced at least in part from adipic acid product produced by the hydrodeoxygenation of a hydrodeoxygenation substrate, and, more particularly, from glucaric acid or derivative thereof.

Other objects and features will become apparent and/or will be pointed out hereinafter.

### DESCRIPTION OF THE PREFERRED EMBODIMENTS

In accordance with the present invention, applicants disclose processes for the chemocatalytic conversion of a glucose source to an adipic acid product.

Further, in accordance with the present invention, applicants disclose processes for the catalytic hydrodeoxygenation of a hydrodeoxygenation substrate comprising glucaric acid and/or derivatives thereof to an adipic acid product. The catalytic hydrodeoxygenation includes reacting, in the presence of a hydrodeoxygenation catalyst (i.e., catalyst suitable for the hydrodeoxygenation reaction) and a halogen source, a hydrodeoxygenation substrate and hydrogen to convert at least a portion of the hydrodeoxygenation substrate to an adipic acid product. The hydrodeoxygenation catalyst of the present invention comprises a d-block metal (i.e., transition metal; groups 3 - 12 of the periodic table) and is hydroxyl, halo, oxo or acyloxy selective, more typically hydroxyl-selective, which increases yield and improves process economics.

The present invention also relates to processes for the catalytic production of glucaric acid from glucose. The process includes reacting glucose with oxygen in the presence of an oxidation catalyst and in the substantial absence of added base, wherein at least 50% of the glucose is converted to glucaric acid. Conducting the oxidation reaction in the substantial absence of added base facilitates product recovery and improves process economics. Further, this reaction can be conducted in the presence of a weak carboxylic acid, such as acetic acid, in which at least a portion of the glucose is solubilized. Moreover, preferred oxidation catalysts and/or oxidation reaction conditions provide yields of glucaric acid in excess of 60%, and up to 65% or more.

In another aspect of the invention, an adipic acid product prepared in accordance with the disclosed processes may be converted, according to processes known in the art, to various other industrially significant chemicals including, for example, adiponitrile, caprolactam, caprolactone, hexamethylene diamine, 1,6-hexanediol, adipate esters, polyamides (e.g., nylon) or polyesters. Thus, adiponitrile, caprolactam, caprolactone, hexamethylene diamine, 1,6-hexanediol, adipate esters, polyamides (e.g., nylon) and polyesters may be prepared from glucose derived from biorenewable sources.

### I. Feedstocks

Glucose can be obtained from various carbohydrate-containing sources including conventional biorenewable sources such as corn grain (maize), wheat, potato, cassava and rice as well as alternative sources such as energy crops, plant biomass, agricultural wastes, forestry residues, sugar processing residues and plant-derived household wastes. More generally, biorenewable sources that may be used in accordance with the present invention include any renewable organic matter that includes a source of carbohydrates such as, for example, switch grass, miscanthus, trees (hardwood and softwood), vegetation, and crop residues (e.g., bagasse and corn stover). Other sources can include, for example, waste materials (e.g., spent paper, green waste, municipal waste, etc.). Carbohydrates such as glucose may be isolated from biorenewable materials using methods that are known in the art. See, for example, Centi and van Santen, Catalysis for Renewables, Wiley-VCH, Weinheim 2007; Kamm, Gruber and Kamm, Biorefineries-Industrial Processes and Products, Wiley-VCH, Weinheim 2006; Shang-Tian Yang, Bioprocessing for Value-Added Products from Renewable Resources New Technologies and Applications, Elsevier B.V. 2007; Furia, Starch in the Food Industry, Chapter 8, CRC Handbook of Food Additives 2nd Edition CRC Press, 1973. See also chapters devoted to Starch, Sugar and Syrups within Kirk-Othmer Encyclopedia of Chemical Technology 5th Edition, John Wiley and Sons 2001. Also, processes to convert starch to glucose are known in the art, see, for example, Schenck, "Glucose and Glucose containing Syrups" in Ullmann's Encyclopedia of Industrial Chemistry, Wiley-VCH 2009. Furthermore, methods to convert cellulose to glucose are known in the art, see, for example, Centi and van Santen, Catalysis for Renewables, Wiley-VCH, Weinheim 2007; Kamm, Gruber and Kamm, Biorefineries-Industrial Processes and Products, Wiley-VCH, Weinheim 2006; Shang-Tian Yang, Bioprocessing for Value-Added Products from Renewable Resources New Technologies and Applications, Elsevier B.V. 2007.

### II. Preparation of Glucaric Acid

In accordance with the present invention, glucose is converted to, for example, glucaric acid. The preparation of glucaric acid can be effected with glucose using oxidation methods that are generally known in the art. See, for example, U.S. Patent No. 2,472,168, which illustrates a method for the preparation of glucaric acid from glucose using a platinum catalyst in the presence of oxygen and a base. Further examples of the preparation of glucaric acid from glucose using a platinum catalyst in the presence of oxygen and a base are illustrated in the Journal of Catalysis Vol. 67, p. 1-13, and p. 14-20 (1981). Other oxidation methods may also be employed, see for example, U.S. Patent Nos. 6,049,004, 5,599,977, and 6,498,269, WO 2008/021054 and J. Chem. Technol. Biotechnol. Vol. 76, p. 186-190 (2001); J. Agr. Food Chem. Vol. 1, p.779-783 (1953); J. Carbohydrate Chem. Vol. 21, p. 65-77 (2002); Carbohydrate Res. Vol. 337, p. 1059-1063 (2002); Carbohydrate Res. 336, p. 75-78 (2001); Carbohydrate Res. Vol. 330, p. 21-29 (2001). However, these processes suffer from economic shortcomings resulting from, among other matters, process yield limitations and the requirement for additional reaction constituents.

Applicants have discovered that glucose may be converted to glucaric acid in high yield by reacting glucose with oxygen (as used herein, oxygen can be supplied to the reaction as air, oxygen-enriched air, oxygen alone, or oxygen with other constituents substantially inert to the reaction) in the presence of an oxidation catalyst and in the absence of added base according to the following reaction: 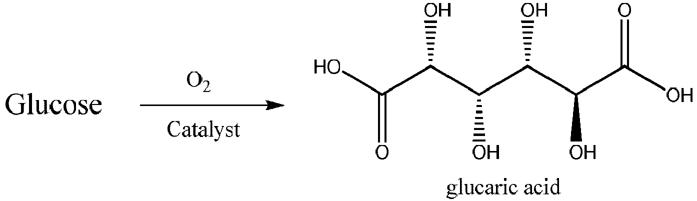 Surprisingly, conducting the oxidation reaction in the absence of added base and in accordance with the reaction conditions set forth herein, does not lead to significant catalyst poisoning effects and catalyst oxidation selectivity is maintained. In fact, catalytic selectivity can be maintained to attain glucaric acid yield in excess of 50%, even 60% and, in some instances, attain yields in excess of 65% or higher. The absence of added base advantageously facilitates separation and isolation of the glucaric acid, thereby providing a process that is more amenable to industrial application, and improves overall process economics by eliminating a reaction constituent. The "absence of added base" as used herein means that base, if present (for example, as a constituent of a feedstock), is present in a concentration which has essentially no effect on the efficacy of the reaction; i.e., the oxidation reaction is being conducted essentially free of added base. It has also been discovered that this oxidation reaction can also be conducted in the presence of a weak carboxylic acid, such as acetic acid, in which glucose is soluble. The term "weak carboxylic acid" as used herein means any unsubstituted or substituted carboxylic acid having a pKa of at least about 3.5, more preferably at least about 4.5 and, more particularly, is selected from among unsubstituted acids such as acetic acid, propionic acid or butyric acid, or mixtures thereof.

It has been further discovered that conducting the oxidation reaction under increased oxygen partial pressures and/or higher oxidation reaction mixture temperatures tends to increase the yield of glucaric acid when the reaction is conducted in the substantial absence of added base.

In these and various other embodiments, the initial pH of the reaction mixture is no greater than about 7, and typically is less than 7 such as, for example, 6 or less when a weak carboxylic acid is used to solubilize at least a portion of the glucose. In accordance with the present invention, the initial pH of the reaction mixture is the pH of the reaction mixture prior to contact with oxygen in the presence of an oxidation catalyst. It is expected that the pH of the reaction mixture after oxygen contact will vary as the reaction proceeds. It is believed that as the concentration of the glucaric acid increases (as the reaction proceeds) the pH will decrease from the initial pH.

Another advantage of the present invention is the essential absence of nitrogen as an active reaction constituent. Typically, nitrogen is employed in known processes as an oxidant such as in the form of nitrate, in many instances as nitric acid. The use of nitrogen in a form in which it is an active reaction constituent, such as nitrate or nitric acid, results in the need for NOₓ abatement technology and acid regeneration technology, both of which add significant cost to the production of glucaric acid from these known processes, as well as providing a corrosive environment which may deleteriously affect the equipment used to carry out the process. By contrast, for example, in the event air or oxygen-enriched air is used in the oxidation reaction of the present invention as the source of oxygen, the nitrogen is essentially an inactive or inert constituent. Thus, for example, in accordance with the present invention, an oxidation reaction employing air or oxygen-enriched air is a reaction conducted essentially free of nitrogen in a form in which it would be an active reaction constituent.

Generally, the temperature of the oxidation reaction mixture is at least about 40°C, more typically 60°C, or higher. In various embodiments, the temperature of the oxidation reaction mixture is from about 40°C to about 150°C, from about 60°C to about 150°C, from about 70°C to about 150°C, from about 70°C to about 140°C, or from about 80°C to about 120°C.

Typically, the partial pressure of oxygen is at least about 15 pounds per square inch absolute (psia) (104 kPa), at least about 25 psia (172 kPa), at least about 40 psia (276 kPa), or at least about 60 psia (414 kPa). In various embodiments, the partial pressure of oxygen is up to about 1000 psia (6895 kPa), or more typically in the range of from about 15 psia (104 kPa) to about 500 psia (3447 kPa).

The oxidation reaction is typically conducted in the presence of a solvent to glucose. Solvents suitable for the oxidation reaction include water and weak carboxylic acids such as acetic acid. Utilization of weak carboxylic acid as a solvent adds cost to the process which cost, as a practical matter, must be balanced against any benefits derived from the use thereof. Thus, suitable solvents for the present invention include water, mixtures of water and weak carboxylic acid, or weak carboxylic acid.

In general, the oxidation reaction can be conducted in a batch, semi-batch, or continuous reactor design using fixed bed reactors, trickle bed reactors, slurry phase reactors, moving bed reactors, or any other design that allows for heterogeneous catalytic reactions. Examples of reactors can be seen in Chemical Process Equipment - Selection and Design, Couper et al., Elsevier 1990. It should be understood that glucose, oxygen, any solvent, and the oxidation catalyst may be introduced into a suitable reactor separately or in various combinations.

Catalysts suitable for the oxidation reaction ("oxidation catalyst") include heterogeneous catalysts, including solid-phase catalysts comprising one or more supported or unsupported metals. In various embodiments, metal is present at a surface of a support (i.e., at one or more surfaces, external or internal). Typically, metal is selected from the group consisting of palladium, platinum, and combinations thereof. Additional other metals may be present, including one or more d-block metals, alone or in combination with one or more rare earth metals (e.g. lanthanides), alone or in combination with one or more main group metals (e.g. Al, Ga, Tl, In, Sn, Pb or Bi). In general, the metals may be present in various forms (e.g., elemental, metal oxide, metal hydroxides, metal ions, etc.). Typically, the metal(s) at a surface of a support may constitute from about 0.25% to about 10%, or from about 1% to about 8%, or from about 2.5% to about 7.5% (e.g., 5%) of the total weight of the catalyst.

In various embodiments, the oxidation catalyst comprises a first metal (M1) and a second metal (M2) at a surface of a support, wherein the M1 metal is selected from the group consisting of palladium and platinum and the M2 metal is selected from the group consisting of d-block metals, rare earth metals, and main group metals, wherein the M1 metal is not the same metal as the M2 metal. In various preferred embodiments, the M1 metal is platinum and the M2 metal is selected from the group consisting of manganese, iron, and cobalt.

The M1:M2 molar ratio may vary, for example, from about 500:1 to about 1:1, from about 250:1 to about 1:1, from about 100:1 to about 1:1, from about 50:1 to about 1:1, from about 20:1 to about 1:1, or from about 10:1 to about 1:1. In various other embodiments, the M1:M2 molar ratio may vary, for example, from about 1:100 to about 1:1, from about 1:50 to about 1:1, from about 1:10 to about 1:1, from about 1:5 to about 1:1, or from about 1:2 to about 1:1.

Moreover, the weight percents of M1 and M2 relative to the catalyst weight may vary. Typically, the weight percent of M1 may range from about 0.5% to about 10%, more preferably from about 1% to about 8%, and still more preferably from about 2.5% to about 7.5% (e.g., about 5%). The weight percent of M2 may range from about 0.25% to about 10%, from about 0.5% to about 8%, or from about 0.5% to about 5%.

In various other embodiments, a third metal (M3) may be added to produce a M1/M2/M3 catalyst wherein the M3 metal is not the same metal as the M1 metal and the M2 metal. In yet other embodiments a fourth metal (M4) may be added to produce a M1/M2/M3/M4 catalyst wherein the M4 metal is not the same metal as the M1 metal, the M2 metal or the M3 metal. The M3 metal and M4 metal may each be selected from the group consisting of d-block metals, rare earth metals (e.g. lanthanides), or main group metals (e.g. Al, Ga, Tl, In, Sn, Pb or Bi).

Suitable catalyst supports include carbon, alumina, silica, ceria, titania, zirconia, niobia, zeolite, magnesia, clays, iron oxide, silicon carbide, aluminosilicates, and modifications, mixtures or combinations thereof. The preferred support materials may be modified using methods known in the art such as heat treatment, acid treatment or by the introduction of a dopant (for example, metal-doped titanias, metal-doped zirconias (e.g., tungstated-zirconia), metal-doped cerias, and metal-modified niobias). Particularly preferred supports are carbon (which may be activated carbon, carbon black, coke or charcoal), alumina, zirconia, titania, zeolite and silica. In various embodiments, the support of the oxidation catalyst is selected from the group consisting of carbon, zirconia, zeolite, and silica.

When a catalyst support is used, the metals may be deposited using procedures known in the art including, but not limited to incipient wetness, ion-exchange, deposition-precipitation, and vacuum impregnation. When two or more metals are deposited on the same support, they may be deposited sequentially or simultaneously. In various embodiments, following metal deposition, the catalyst is dried at a temperature of at least about 50°C, more typically at least about 120°C for a period of time of at least about 1 hour, more typically 3 hours or more. In these and other embodiments, the catalyst is dried under sub-atmospheric pressure conditions. In various embodiments, the catalyst is reduced after drying (e.g., by flowing 5% H₂ in N₂ at 350 °C for 3 hours). Still further, in these and other embodiments, the catalyst is calcined, for example, at a temperature of at least about 500°C for a period of time (e.g., at least about 3 hours).

The reaction product of the oxidation step will, as described above, yield glucaric acid in considerable and heretofore unexpected fraction, but may also yield derivatives thereof, such as glucarolactones. These glucarolactones, like glucaric acid, constitute hydrodeoxygenation substrate which is particularly amenable to the production of adipic acid product as hereinafter described. Glucarolactones which may be present in the reaction mixture resulting from the oxidation step include mono and di-lactones such as D-glucaro-1,4-lactone, D-glucaro-6,3- lactone, and D-glucaro-1,4:6,3-dilactone. One advantage of higher concentrations of glucarolactones is further improvement in the economics of the hydrodeoxygenation step resulting from a reduction in the amount of water produced.

Glucaric acid produced in accordance with the above may be converted to various other glucaric acid derivatives, such as salts, esters, ketones, and lactones. Methods to convert carboxylic acids to such derivatives are known in the art, see, for example, Wade, Organic Chemistry 3rd ed, Prentice Hall 1995.

### III. Preparation of an Adipic Acid Product

In accordance with the present invention, an adipic acid product may be prepared by chemocatalytic conversion of a glucose source. In various embodiments, preparation of an adipic acid product includes chemocatalytic conversion of a glucose source to glucaric acid. In these and other embodiments, a hydrodeoxygenation substrate comprising at least a portion of the glucaric acid or derivatives thereof is converted to an adipic acid product. Derivatives of glucaric acid include compounds as defined below.

The hydrodeoxygenation substrate comprises a compound of the formula I: 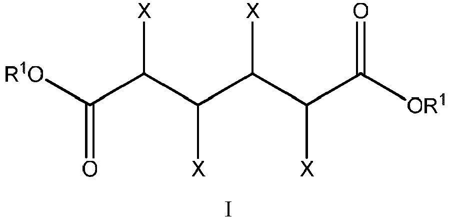 wherein X is independently hydroxyl, oxo, halo, acyloxy or hydrogen provided that at least one X is not hydrogen; R¹ is independently a salt-forming ion, hydrogen, or hydrocarbyl; or a mono- or di-lactone thereof.

As used herein, the term "hydrocarbyl" refers to hydrocarbyl moieties containing 1 to about 18 carbon atoms, including branched or unbranched, and saturated or unsaturated species. Preferred hydrocarbyl can be selected from the group consisting of alkyl, alkylene, cycloalkyl, cycloalkylalkyl, and aryl.

Salt forming ions include, without limitation, for example ammonium ions and metal ions (e.g., alkali and alkaline earth metals). When R¹ is a salt forming ion (i.e., a cation), the carboxyl group may be considered to be anion (i.e., carboxylate anion).

In various embodiments, the hydrodeoxygenation substrate comprises a compound of formula I, wherein X is hydroxyl and R¹ is independently a salt-forming ion, hydrogen, or hydrocarbyl.

As shown in formula I, the hydrodeoxygenation substrate contains a six carbon chain comprising four chiral centers. As a result, several stereoisomers are possible. However, the preferred hydrodeoxygenation substrate comprises glucaric acid.

The hydrodeoxygenation substrate may also contain various ketones. For example, not wishing to be bound by theory, when glucaric acid is further oxidized, ketones such as 2-keto-glucaric acid (2,3,4-trihydroxy-5-oxohexanedioic acid) and 3-keto-glucaric acid (2,3,5-trihydroxy-4-oxohexanedioic acid) may be formed.

The hydrodeoxygenation substrate may comprise various lactones derived from glucaric acid. For example, not wishing to be bound by theory, it is believed that various mono- and di-lactones are present in equilibrium with glucaric acid in aqueous solution, including, for example, D-glucaro-1,4-lactone, D-glucaro-6,3-lactone, and D-glucaro-1,4:6,3-dilactone. Moreover, processes have been developed to quantitatively convert glucaric acid or a salt thereof in solution to one or more lactones and recover a substantially pure lactone stream. For example, see "Convenient Large-Scale Synthesis of D-Glucaro-1,4:6,3-dilactone" Gehret et al., J. Org. Chem., 74 (21), pp. 8373-8376 (2009). Also, lactones such as L-*threo*-4-deoxy-hex-4-enaro-6,3-lactone and L-*erythro*-4-deoxy-hex-4-enaro-6,3-lactone may form from the thermal decomposition of D-Glucaro-1,4:6,3-dilactone.

Therefore, in various embodiments, the hydrodeoxygenation substrate comprises D-glucaro-1,4-lactone. In these and other embodiments, the hydrodeoxygenation substrate comprises D-glucaro-6,3-lactone. Still further, in these and other embodiments, the hydrodeoxygenation substrate comprises D-glucaro-1,4:6,3-dilactone. In these and other embodiments, the hydrodeoxygenation substrate comprises L-*threo*-4-deoxy-hex-4-enaro-6,3-lactone. Still even further, in these and other embodiments, the hydrodeoxygenation substrate comprises L-*erythro*-4-deoxy-hex-4-enaro-6,3-lactone.

Also, in accordance with the present invention, an adipic acid product (formula II) may be prepared by reacting, in the presence of a hydrodeoxygenation catalyst and a halogen source, a hydrodeoxygenation substrate (formula I) and hydrogen, according to the following reaction: 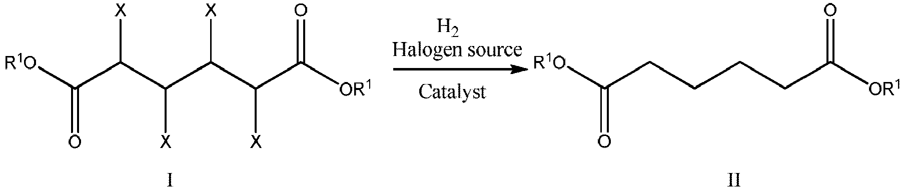 wherein X and R¹ are defined as described above.

In preferred embodiments, the adipic acid product (formula II) comprises adipic acid.

In the above reaction, a hydrodeoxygenation substrate is converted to an adipic acid product by catalytic hydrodeoxygenation in which carbon-hydroxyl groups are converted to carbon-hydrogen groups. In various embodiments, the catalytic hydrodeoxygenation is hydroxyl-selective wherein the reaction is completed without substantial conversion of the one or more other non-hydroxyl functional group of the substrate.

In accordance with the present invention, a hydrodeoxygenation substrate is catalytically hydrodeoxygenated in the presence of hydrogen, a halogen source and a hydrodeoxygenation catalyst. Without being bound by theory, it is believed that during this reaction the hydrodeoxygenation substrate is halogenated with the halogen source, to form a halogenated intermediate containing a carbon-halogen bond (e.g., a secondary alcohol group on the glucaric acid is converted to a halide to produce an alkyl halide). The carbon-halogen bond of the halogenated intermediate is believed to be converted to a carbon-hydrogen bond via one or more of the following pathways. In the first pathway, the halogenated intermediate reacts with hydrogen in the presence of the hydrodeoxygenation catalyst leading to the formation of a carbon-hydrogen bond along with the generation of hydrohalic acid. In the second pathway, the halogenated intermediate undergoes a dehydrohalogenation reaction to form an olefin intermediate and hydrohalic acid. The olefin is further reduced in the presence of the hydrodeoxygenation catalyst leading to the formation of a carbon-hydrogen bond (or the olefin may be an enol form of a ketone which can interconvert to a keto form which can reduce to an alcohol group which can undergo further hydrodeoxygenation). Effecting the reaction pursuant to the above described first and second pathways generates hydrohalic acid as a by-product, which is available for further reaction. In the third pathway, the halogenated intermediate reacts with hydrohalic acid leading to the formation of a carbon-hydrogen bond along with the formation of molecular halogen (or interhalogen). Effecting the reaction pursuant to the third pathway generates molecular halogen as a by-product, which is available for further reaction. One or more of the various pathways described above may occur concurrently.

It should be recognized that the hydrodeoxygenation reaction can be conducted by first forming and optionally purifying or isolating these various intermediates formed by combining a hydrodeoxygenation substrate and a halogen source and subsequently reacting the intermediate with hydrogen in the presence of the hydrodeoxygenation catalyst and optionally in the absence of any halogen source.

The hydrodeoxygenation substrate is halogenated with hydrohalic acid to form a halogenated intermediate (e.g., an alkyl halide).

Halogen sources include hydrohalic acids (e.g., HCl, HBr, HI and mixtures thereof; preferably HBr and/or HI). In more preferred embodiments, the halogen source is hydrogen bromide.

Generally, the molar ratio of halogen to the hydrodeoxygenation substrate is about equal to or less than about 1. In various embodiments, the mole ratio of halogen to the hydrodeoxygenation substrate is typically from about 1:1 to about 0.1:1, more typically from about 0.7:1 to about 0.3:1, and still more typically about 0.5:1.

Generally, the reaction allows for recovery of the halogen source and catalytic quantities (where molar ratio of halogen to the hydrodeoxygenation substrate is less than about 1) of halogen can be used, recovered and recycled for continued use as a halogen source.

Generally, the temperature of the hydrodeoxygenation reaction mixture is at least about 20°C, typically at least about 80°C, and more typically at least about 100°C. In various embodiments, the temperature of the hydrodeoxygenation reaction is conducted in the range of from about 20°C to about 250°C, from about 80°C to about 200°C, more preferably from about 120°C to about 180°C, and still more preferably between about 140°C and 180°C.

Typically, the partial pressure of hydrogen is at least about 25 psia (172 kPa), more typically at least about 200 psia (1379 kPa) or at least about 400 psia (2758 kPa). In various embodiments, the partial pressure of hydrogen is from about 25 psia (172 kPa) to about 2500 psia (17237 kPa), from about 200 psia (1379 kPa) to about 2000 psia (13790 kPa), or from about 400 psia (2758 kPa) to about 1500 psia (10343 kPa).

The hydrodeoxygenation reaction is typically conducted in the presence of a solvent. Solvents suitable for the selective hydrodeoxygenation reaction include water and carboxylic acids, amides, esters, lactones, sulfoxides, sulfones and mixtures thereof. Preferred solvents include water, mixtures of water and weak carboxylic acid, and weak carboxylic acid. A preferred weak carboxylic acid is acetic acid.

In general, the reaction can be conducted in a batch, semi-batch, or continuous reactor design using fixed bed reactors, trickle bed reactors, slurry phase reactors, moving bed reactors, or any other design that allows for heterogeneous catalytic reactions. Examples of reactors can be seen in Chemical Process Equipment - Selection and Design, Couper et al., Elsevier 1990. It should be understood that the hydrodeoxygenation substrate, halogen source, hydrogen, any solvent, and the hydrodeoxygenation catalyst may be introduced into a suitable reactor separately or in various combinations.

In various preferred embodiments, the hydrodeoxygenation catalyst is heterogeneous, but a suitable homogeneous catalyst may be employed. In these and various other preferred embodiments the hydrodeoxygenation catalyst comprises a solid-phase heterogeneous catalyst in which one or more metals is present at a surface of a support (i.e., at one or more surfaces, external or internal). Preferred metals are d-block metals which may be used alone, in combination with each other, in combination with one or more rare earth metals (e.g. lanthanides), and in combination with one or more main group metals (e.g., Al, Ga, Tl, In, Sn, Pb or Bi). Preferred d-block metals are selected from the group consisting of cobalt, nickel, ruthenium, rhodium, palladium, osmium, iridium, platinum and combinations thereof. More preferred d-block metals are selected from the group consisting of ruthenium, rhodium, palladium, platinum, and combinations thereof. In certain preferred embodiments, the catalyst comprises platinum. In general, the metals may be present in various forms (e.g., elemental, metal oxide, metal hydroxides, metal ions etc.). Typically, the metal(s) at a surface of a support may constitute from about 0.25% to about 10%, or from about 1% to about 8%, or from about 2.5% to about 7.5% (e.g., 5%) of the catalyst weight.

In various embodiments, the catalyst comprises two or more metals. For example, two of more metals (M1 and M2) may be co-supported on or within the same support (e.g., as a mixed-metal catalyst on silica; M1/M2/Silica catalyst), or they may be supported on different support materials. In various embodiments the hydrodeoxygenation catalyst comprises a first metal (M1) and a second metal (M2) at a surface of a support, wherein the M1 metal comprises a d-block metal and the M2 metal is selected from the group consisting of d-block metals, rare earth metals, and main group metals, wherein the M1 metal is not the same metal as the M2 metal. In various embodiments, the M1 metal is selected from the group consisting of cobalt, nickel, ruthenium, rhodium, palladium, osmium, iridium, and platinum. In more preferred embodiments, the M1 metal is selected from the group consisting of ruthenium, rhodium, palladium, and platinum. In various embodiments, the M2 metal is selected from the group consisting of titanium, vanadium, chromium, manganese, iron, cobalt, nickel, copper, molybdenum, ruthenium, rhodium, palladium, silver, tungsten, iridium, platinum, and gold. In more preferred embodiments, the M2 metal is selected from the group consisting of molybdenum, ruthenium, rhodium, palladium, iridium, platinum, and gold.

In more preferred embodiments, the M1 metal is selected from the group of platinum, rhodium and palladium, and the M2 metal is selected from the group consisting of ruthenium, rhodium, palladium, platinum, and gold. In certain more preferred embodiments, M1 is platinum and M2 is rhodium.

In various embodiments, the M1:M2 molar ratio may vary, for example, from about 500:1 to about 1:1, from about 250:1 to about 1:1, from about 100:1 to about 1:1, from about 50:1 to about 1:1, from about 20:1 to about 1:1, or from about 10:1 to about 1:1. In various other embodiments, the M1:M2 molar ratio may vary, for example, from about 1:100 to about 1:1, from about 1:50 to about 1:1, from about 1:10 to about 1:1, from about 1:5 to about 1:1, or from about 1:2 to about 1:1.

Moreover, in various embodiments, the weight percents of M1 and M2 relative to the total catalyst weight may vary. Typically, the weight percent of M1 may range from about 0.5% to about 10%, more preferably from about 1% to about 8%, and still more preferably from about 2.5% to about 7.5% (e.g., about 5%). The weight percent of M2 may range from about 0.25% to about 10%, from about 0.5% to about 8%, or from about 0.5% to about 5%.

In various other embodiments, a third metal (M3) may be added to produce a M1/M2/M3 catalyst wherein the M3 metal is not the same metal as the M1 metal and the M2 metal. In other embodiments a fourth metal (M4) may be added to produce a M1/M2/M3/M4 catalyst wherein the M4 metal is not the same metal as the M1 metal, the M2 metal or the M3 metal. M3 and M4 may each be selected from the group consisting of d-block metals, rare earth metals (e.g. lanthanides), or main group metals (e.g. Al, Ga, Tl, In, Sn, Pb or Bi).

Preferred catalyst supports include carbon, alumina, silica, ceria, titania, zirconia, niobia, zeolite, magnesia, clays, iron oxide, silicon carbide, aluminosilicates, and modifications, mixtures or combinations thereof. The preferred supports may be modified through methods known in the art such as heat treatment, acid treatment, the introduction of a dopant (for example, metal-doped titanias, metal-doped zirconias (e.g. tungstated zirconia), metal-doped cerias, and metal-modified niobias). In various preferred embodiments, the hydrodeoxygenation catalyst support is selected from the group consisting of silica, zirconia and titania. In certain preferred embodiments, a catalyst comprising platinum and rhodium is on a support comprising silica.

When a catalyst support is used, the metals may be deposited using procedures known in the art including, but not limited to incipient wetness, ion-exchange, deposition-precipitation and vacuum impregnation. When the two or more metals are deposited on the same support, they may be deposited sequentially, or simultaneously. In various embodiments, following metal deposition, the catalyst is dried at a temperature of at least about 50°C, more typically at least about 120°C or more for a period of time of at least about 1 hour, more typically at least about 3 hours or more. In these and other embodiments, the catalyst is dried under sub-atmospheric conditions. In various embodiments, the catalyst is reduced after drying (e.g., by flowing 5% H₂ in N₂ at 350 °C for 3 hours). Still further, in these and other embodiments, the catalyst is calcined, for example, at a temperature of at least about 500°C for a period of time (e.g., at least about 3 hours).

Without being bound by theory not expressly recited in the claims, catalysts mixtures (co-catalysts or mixed metal catalysts) containing more than one metal may affect separate steps of the mechanistic reaction pathway.

An adipic acid product may be recovered from the hydrodeoxygenation reaction mixture by one or more conventional methods known in the art including, for example, solvent extraction, crystallization or evaporative processes.

### IV. Downstream Chemical Products

Various methods are known in the art for conversion of adipic acid to downstream chemical products or intermediates including adipate esters, polyesters, adiponitrile, hexamethylene diamine (HMDA), caprolactam, caprolactone, 1,6-hexanediol, aminocaproic acid, and polyamide such as nylons. For conversions from adipic acid, see for example, without limitation, U.S. Patent Nos. 3,671,566, 3,917,707, 4,767,856, 5,900,511, 5,986,127, 6,008,418, 6,087,296, 6,147,208, 6,462,220, 6,521,779, 6,569,802, and Musser, "Adipic Acid" in Ullmann's Encyclopedia of Industrial Chemistry, Wiley-VCH, Weinheim, 2005.

In various embodiments, an adipic acid product is converted to adiponitrile wherein the adipic acid product is prepared in accordance with the present invention. Adiponitrile can be used industrially for the manufacture of hexamethylene diamine, see Smiley, "Hexamethylenediamine" in Ullman's Encyclopedia of Industrial Chemistry, Wiley-VCH 2009. Therefore, in further embodiments, an adipic acid product is converted to hexamethylene diamine wherein the adipic acid product is prepared in accordance with the present invention.

Adipic acid is useful in the production of polyamides, such as nylon 6,6 and nylon 4,6. See, for example, U.S. Patent No. 4,722,997, and Musser, "Adipic Acid" in Ullmann's Encyclopedia of Industrial Chemistry, Wiley-VCH, Weinheim, 2005. The hexamethylene diamine formed from an adipic acid product prepared in accordance with the present invention can likewise be further used for the preparation of polyamides such as nylon 6,6 and nylon 6,12. See, for example Kohan, Mestemacher, Pagilagan, Redmond, "Polyamides" in Ullmann's Encyclopedia of Industrial Chemistry, Wiley-VCH, Weinheim, 2005.

Accordingly, adipic acid and a polymer precursor derived from an adipic acid product (e.g., hexamethylene diamine) may be reacted to produce a polyamide, wherein the adipic acid product is prepared in accordance with the present invention. Polymer precursor, as used herein, refers to a monomer which can be converted to a polymer (or copolymer) under appropriate polymerization conditions. In various embodiments, the polyamide comprises nylon 6,6. In these embodiments, nylon 6,6 is produced by reacting an adipic acid product with a polymer precursor derived from an adipic acid product, wherein the polymer precursor comprises hexamethylene diamine. In these embodiments, hexamethylene diamine may be prepared by converting an adipic acid product to adiponitrile which then may be converted to hexamethylene diamine, wherein the adipic acid product is prepared in accordance with the present invention.

In other embodiments, an adipic acid product is converted to caprolactam wherein the adipic acid product is prepared in accordance with the present invention. The caprolactam formed can be further used for the preparation of polyamides by means generally known in the art. Specifically, caprolactam can be further used for the preparation of nylon 6. See, for example Kohan, Mestemacher, Pagilagan, Redmond, "Polyamides" in Ullmann's Encyclopedia of Industrial Chemistry, Wiley-VCH, Weinheim, 2005.

In various embodiments, nylon 6 is produced by reacting caprolactam derived from an adipic acid product prepared in accordance with the present invention.

In other embodiments, adipic acid and a polymer precursor may be reacted to produce a polyester, wherein the adipic acid product is prepared in accordance with the present invention.

In other embodiments, an adipic acid product is converted to 1,6-hexanediol wherein the adipic acid product is prepared in accordance with the present invention. 1,6-hexanediol is a valuable chemical intermediate used in the production of polyesters and polyurethanes. Accordingly, in various embodiments, polyester may be prepared by reacting adipic acid and 1,6-hexandiol derived from an adipic acid product, prepared in accordance with the present invention.

In various embodiments a salt of adipic acid may be produce wherein the process comprises reacting adipic acid with hexamethylene diamine, thereby forming the salt, wherein adipic acid is prepared in accordance with the present invention.

In view of the above, it will be seen that the several objects of the invention are achieved and other advantageous results attained.

### EXAMPLES

The following non-limiting examples are provided to further illustrate the present invention.

Reactions were conducted in 1 mL glass vials housed in a pressurized vessel in accordance with the procedures described in the examples below. Product yields were determined using a Dionex ICS-3000 Chromatography system. In Example 1, the products were first separated on an Ionpac® AS11-HC column and then quantified by conductivity detection through comparison with calibration standards. In Example 2, the products were first separated on an Acclaim® Organic Acid column and then quantified by a UV detector through comparison with calibration standards.

### EXAMPLE 1: GLUCOSE TO GLUCARIC ACID

Several catalysts were obtained from commercial vendors: Johnson Matthey 5% Pt/C (three examples; JM-23 [B103032-5, Lot #C-9090]; JM-25 [B103014-5, Lot #C9230]; and JM-27 [B-501032-5, Lot #C-9188]), Johnson Matthey 5% Pt/Al₂O₃ (two examples; JM-32 [B301013-5, Lot #C8959] and JM-33 [B301099-5, Lot #C9218]), and BASF Escat 2351 5% Pt/SiO₂ [Lot #A4048107]; and 1.5% Au/TiO₂ [Süd Chemie 02-10]. Other catalysts were prepared in accordance with the procedure described herein.

### Preparation of Supported Platinum Catalysts

Multiple portions of suitably concentrated aqueous Pt(NO₃)₂ solutions (Heraeus) were added to the appropriate support (wherein the total combined volume of the Pt(NO₃)₂ solutions were matched to equal to the pore volume of the chosen support) with agitation between additions. Post impregnation, the product was dried in a furnace at 120 °C for 12 hours, Material for catalyst testing was prepared by reduction under flowing 5 vol.% H₂ in N₂ for 3 hours at either 200 °C or 350 °C. Note that this procedure was used for all supports except carbon. See the later description for the preparation of a Pt/Carbon catalyst.

### Preparation of Pt/M2/Support Catalysts (M2 = Mn, Co, Fe, Re, Cu)

Approximately 7 - 8 mg of dried supported platinum catalyst (taken post drying but prior to reduction) was dispensed into an 8 x 12 array containing 1 mL glass vials. To select vials within the array, 6 - 7 µl (where the total addition volume was matched to equal to the pore volume of the support weighed into the vial) of suitably concentrated M2 stock solutions were added (M2 = Mn, Fe, Co, Re, Cu obtained from Strem or Sigma-Aldrich, see Table 1). Post M2 addition, the mixtures were agitated via a multi-tube vortexer to impregnate the supports. Post impregnation, the glass vial arrays of Pt/M2/Support catalysts were dried in a furnace at 120 °C for 1 hour, followed by calcination at 500 °C for 3 hours followed by reduction under flowing 5 vol. % H₂ in N₂ at either 200 °C or 350 °C for 3 hours. Note that this procedure was used to prepare all Pt/M2/Support catalysts with the exception of the 1.5 % Pt/1.5% Au/Titania catalyst. In this case Pt(NO₃)₂ solution was added to a dried sample of the commercial 1.5% Au/Titania catalyst [Süd Chemie 02-10] (wherein the total volume of the Pt(NO₃)₂ volume was matched to equal to the pore volume of the catalyst) with agitation, whereupon the material was dried in a furnace at 120 °C for 1 hour, followed by reduction under flowing 5 vol. % H₂ in N₂ at 350 °C for 3 hours.

### Preparation of 4 wt.% Pt/Carbon Catalyst

Multiple portions of suitably concentrated aqueous Pt(NO₃)₂ solution (Heraeus) were added to 2 g of dried Degussa HP-160 furnace black carbon (3.94 mL total addition volume) with agitation between additions. Post impregnation, the 4 wt.% Pt/Carbon was dried under vacuum for one hour at 50°C, followed by reduction under flowing 5 vol.% H₂ in N₂ for three hours at 350°C.

### Glucose to Glucaric Acid Reactions

Catalysts were dispensed into 1mL vials within a 96-well reactor insert (Symyx Solutions). The reaction substrate was D-glucose (Sigma-Aldrich, 0.552M in water). To each vial was added 250 µL of glucose solution. The vials were each covered with a Teflon pin-hole sheet, a silicone pin-hole mat and steel gas diffusion plate (Symyx Solutions). The reactor insert was placed in a pressure vessel and charged three times with oxygen to 791 kPa (100 psig) with venting after each pressurization step. The reactor was then charged to 618 kPa (75 psig) with oxygen, or to 3955 kPa (500 psig) with air, closed and placed on a shaker, heated at the designated temperature for the specified reaction time. After the reaction time had elapsed shaking was stopped and the reactor cooled to room temperature whereupon the reactors were vented. Samples for ion-chromatography (IC) analysis were prepared by adding to each reaction vial 750 µL of a 1.067 wt.% citric acid solution (as internal standard) then the plate was covered and mixed followed by centrifugation to separate catalyst particles. Each reaction sample was further diluted by performing two 20-fold dilutions then analyzed by Ion Chromatography. In some instances, HCl was used as alternative internal standard through the addition of 100 µL of 50 ppm solution during the second 20-fold dilution. The results are presented in Table 1.

**Table 1.**

| | **Catalyst (wt.% M2 wt.% Pt/Support)** | **M1 Precursor** | **M2 Precursor** | **Temp. (°C)** | **Time (Hours)** | **Catalyst Amount (mg)** | **Glucaric Acid Yield (%)** |
|---|---|---|---|---|---|---|---|
| 1 | 0.06% Mn 4% Pt / Silica Davisil 635 | Pt(NO₃)₂ | Mn(NO₃)₂ | 80 | 5 | 7 | 38 |
| 2 | 0.06% Fe 4% Pt / Silica Davisil 635 | Pt(NO₃)₂ | Fe(NO₃)₃ | 80 | 5 | 8 | 28 |
| 3 | 0.06% Co 4% Pt / Silica Davisil 635 | Pt(NO₃)₂ | Co(NO₃)₂ | 80 | 5 | 8 | 34 |
| 4 | 4% Pt / Silica Davisil 635 | Pt(NO₃)₂ | None | 80 | 5 | 8 | 34 |
| 5 | 4% Pt / Silica 5 µm Cariact | Pt(NO₃)₂ | None | 90 | 5 | 8 | 50 |
| 6 | 4% Pt / Silica 5 µm Cariact | Pt(NO₃)₂ | None | 90 | 8 | 8 | 66 |
| 7 | 4% Pt / Silica Merck 10180 | Pt(NO₃)₂ | None | 90 | 5 | 8 | 40 |
| 8 | 1.91% Re 4% Pt / Silica Merck 10180 | Pt(NO₃)₂ | HReO₄ | 90 | 5 | 8 | 39 |
| 9 | 0.65% Cu 4% Pt / Silica Merck 10180 | Pt(NO₃)₂ | Cu(NO₃)₂ | 90 | 5 | 8 | 39 |
| 10 | 0.10% Mo 4% Pt/Silica Merck 10180 | Pt(NO₃)₂ | (NH₄)₆Mo₇O₂₄ | 90 | 5 | 8 | 38 |
| 11 | 4% Pt / Carbon Degussa HP-160 | Pt(NO₃)₂ | None | 80 | 5 | 8 | 53 |
| 12 | 4% Pt / Carbon Degussa HP-160 | Pt(NO₃)₂ | None | 90 | 8 | 8 | 60 |
| 13 | 5% Pt / C [JM-23] | | None | 80 | 5 | 10 | 52 |
| 14 | 5% Pt / C [JM-25] | | None | 80 | 5 | 10 | 57 |
| 15 | 5% Pt / C [JM-27] | | None | 80 | 5 | 10 | 57 |
| 16 | 5% Pt / Al₂O₃ [JM-32] | | None | 80 | 5 | 10 | 23 |
| 17 | 5% Pt / Al₂O₃ [JM-33] | | None | 80 | 5 | 10 | 31 |
| 18 | 5% Pt / SiO₂ [BASF Escat 2351] | Pt(NO₃)₂ | None | 80 | 5 | 10 | 15 |
| 19 | 8% Pt / Zirconia Daiichi Kigenso Z-1044 | Pt(NO₃)₂ | None | 90 | 5 | 8 | 52 |
| 20 | 8% Pt / Zirconia Daiichi Kigenso Z-1628 | Pt(NO₃)₂ | None | 90 | 5 | 8 | 59 |
| 21 | 8% Pt / Zirconia Ceria Daiichi Kigenso Z-1006 | Pt(NO₃)₂ | None | 90 | 5 | 8 | 54 |
| 22 | 8% Pt / Ceria Daiichi Kigenso Z-1627 | Pt(NO₃)₂ | None | 90 | 5 | 8 | 17 |
| ^{b}23 | ^{a}4% Pt / Zeolite Zeolyst CP 811C-300 | Pt(NO₃)₂ | None | 100 | 5 | 8 | 39 |
| ^{b}24 | ^{a}4% Pt / Titania NorPro ST 61120 | Pt(NO₃)₂ | None | 100 | 5 | 8 | 30 |
| ^{b}24 | 1.5% Pt 1.5% Au / Titania [Süd Chemie 02-10] | | Pt(NO₃)₂ | 100 | 5 | 8 | 55 |
| ^{b}25 | 4% Pt 4% Au / Titania NorPro ST 61120 | AuCl₃ | Pt(NO₃)₂ | 100 | 5 | 8 | 32 |

| | | | | | | | |
|---|---|---|---|---|---|---|---|
| ^{a}These catalysts were calcined at 500 °C for 3 hours prior to reduction. ^{b}These reactions were run under 3955 kPa (500 psig) of air, all other reactions in Table 1 were run under 618 kPa (75 psig) of O₂. Catalysts in examples 4-7, 11-12 were reduced at 200 °C under flowing 5 vol.% H₂ in N₂ for 3 hours. Catalysts in examples 1-3, 8-10, 19-25 were reduced at 350 °C under flowing 5 vol.% H₂ in N₂ for 3 hours. Commercial catalysts in examples 13-18 were screened directly. | | | | | | | |

### EXAMPLE 2: GLUCARIC ACID TO ADIPIC ACID

### Preparation of M1/Support Catalysts (M1 = Ru, Rh, Pd, Pt)x

2 g of dried 5 µm Silica Cariact (Fuji Silysia) or 45 µm Titania NorPro ST 61120 (Saint-Gobain) was weighed into vials. Suitably concentrated M1 stock solutions (M1 = Ru, Rh, Pd, Pt) were prepared from concentrated acidic stock solutions purchased from Heraeus (see Table 1). For each M1, multiple additions of the dilute M1 stock solution were added to the Support (Silica pore volume = 0.7 mL/g, Titania NorPro = 0.45 mL/g) until a total volume of 1.4 ml (Silica) or 0.9 mL (Titania) was reached. After each addition, the mixtures were agitated to impregnate the support. Post impregnation, the X wt.% M1/Support mixtures (X = 2 - 5) were dried in a furnace at 120 °C for 12 hours.

### Preparation of Ml/M2/Support Catalysts (M2 = Ru, Rh, Pd, Ir, Pt, Au, Mo)

7 - 12 mg of dried X wt.% M1/Support (M1 = Ru, Rh, Pd Pt) (X = 2 - 5) were dispensed into 8 x 12 arrays containing 1 mL glass vials. To select vials within the array, 3 - 8 µl (where the total addition volume was matched to equal to the pore volume of the dried X wt.% M1/Support catalysts weighed into the vial) of suitably concentrated M2 stock solutions were added (M2 = Ru, Rh, Pd, Ir, Pt, Au (obtained from Heraeus), and Mo (obtained from Strem), see Table 1). Post M2 addition, the mixtures were agitated via a multi-tube vortexer to impregnate the supports. Post impregnation, the glass vial arrays of M1/M2/Support catalysts were dried in a furnace at 120 °C for 1 hour, followed by calcination at 500 °C for 3 hours. Upon cooling the arrays of catalysts were stored in a dessicator until used.

### Preparation of M1/M2/Support Catalysts by Coimpregnation (M1/M2 = Rh, Pd, Pt).

7 - 12 mg of silica support (Davisil 635 W.R.Grace & Co.) were dispensed into 8 x 12 arrays containing 1 mL glass vials. Supports were dried at 120 °C for 12 hours prior to use. To select vials within the array, 6 - 11 µl (where the total addition volume was matched to equal to the pore volume of the Support weighed into the vial) of suitably concentrated premixed M1/M2 stock solutions were added (M1/M2 =Rh, Pd, Pt) (obtained from Heraeus), Post metal addition, the mixtures were agitated via a multi-tube vortexer to impregnate the supports. Post impregnation, the glass vial arrays of M1/M2/Support catalysts were dried in a furnace at 120 °C for 1 hour, followed by calcination at 500 °C for 3 hours. Upon cooling, the arrays of catalysts were stored in a dessicator until used.

### Glucaric Acid to Adipic Acid Reactions.

The arrays of catalysts were transferred to 1 mL glass vials within a 96-well reactor insert (Symyx Solutions). Each vial within each array received a glass bead and 250 µL of 0.2 M Glucaric Acid (prepared from calcium glucarate) (Sigma-Adrich), 0.1 M HBr (examples 1-37; Sigma-Aldrich) or 0.2 M HBr (example 38; Sigma-Aldrich) in Acetic Acid (Sigma-Aldrich). Upon solution addition, the arrays of vials were covered with a Teflon pin-hole sheet, a silicone pin-hole mat and steel gas diffusion plate (Symyx Solutions). The reactor insert was placed in a pressure vessel pressurized and vented 3 times with nitrogen and 3 times with hydrogen before being pressurized with hydrogen to 4997 kPa (710 psig), heated to 140 °C (examples 1-37) or 160 °C (example 38) and shaken for 3 hours. After 3 hours the reactors were cooled, vented and purged with nitrogen. 750 µl of water was then added to each vial. Following the water addition, the arrays were covered and shaken to ensure adequate mixing. Subsequently, the covered arrays were placed in a centrifuge to separate the catalyst particles. Each reaction sample was then diluted 2-fold with water to generate a sample for analysis by HPLC. The results are presented in Table 2.

**Table 2.**

| | **Catalyst (wt. % M2 wt. % M1 / Support)** | **M1 Precursor** | **M2 Precursor** | **Catalyst Amount (mg)** | **Adipic Acid Yield (%)** |
|---|---|---|---|---|---|
| 1 | 0.5% Pd 5% Rh / Silica 5 µm Cariact | Rh(NO₃)₃ | Pd(NO₃)₂ | 8 | 51 |
| 2 | 0.5% Pd 5% Rh / Silica 5 µm Cariact | Rh(NO₃)₃ | Pd(NO₃)₂ | 8 | 53 |
| 3 | 0.5% Ru 5% Pd / Silica 5 µm Cariact | Pd(NO₃)₂ | ^{a}Ru(NO)(NO₃)ₓ(OH)_{y} | 8 | 74 |
| 4 | 0.5% Ru 5% Pd / Silica 5 µm Cariact | Pd(NO₃)₂ | ^{a}Ru(NO)(NO₃)ₓ(OH)_{y} | 8 | 77 |
| 5 | 0.5% Ru 5% Rh / Silica 5 µm Cariact | Rh(NO₃)₃ | ^{a}Ru(NO)(NO₃)ₓ(OH)_{y} | 10 | 50 |
| 6 | 0.5% Ru 5% Rh / Silica 5 µm Cariact | Rh(NO₃)₃ | ^{a}Ru(NO)(NO₃)ₓ(OH)_{y} | 8 | 57 |
| 7 | 1% Ir 5% Rh / Silica 5 µm Cariact | Rh(NO₃)₃ | H₂IrCl₆.H₂O | 8 | 53 |
| 9 | 2.5% Pd 5% Rh / Silica 5 µm Cariact | Rh(NO₃)₃ | Pd(NO₃)₂ | 8 | 50 |
| 10 | 2.5% Pd 5% Rh / Silica 5 µm Cariact | Rh(NO₃)₃ | Pd(NO₃)₂ | 13 | 50 |
| 11 | 2.5% Ru 5% Pd / Silica 5 µm Cariact | Pd(NO₃)₂ | ^{a}Ru(NO)(NO₃)ₓ(OH)_{y} | 8 | 63 |
| 12 | 2.5% Ru 5% Pd / Silica 5 µm Cariact | Pd(NO₃)₂ | ^{a}Ru(NO)(NO₃)ₓ(OH)_{y} | 8 | 63 |
| 13 | 2.5% Ru 5% Rh / Silica 5 µm Cariact | Rh(NO₃)₃ | ^{a}Ru(NO)(NO₃)ₓ(OH)_{y} | 11 | 51 |
| 14 | 2.5% Ru 5% Rh / Silica 5 µm Cariact | Rh(NO₃)₃ | ^{a}Ru(NO)(NO₃)ₓ(OH)_{y} | 8 | 54 |
| 15 | 5% Ir 5% Rh / Silica 5 µm Cariact | Rh(NO₃)₃ | H₂IrCl₆.H₂O | 8 | 52 |
| 16 | 5% Pt 5% Pd / Silica 5 µm Cariact | Pd(NO₃)₂ | Pt(NO₃)₂ | 8 | 54 |
| 17 | ^{b}5% Rh/ Silica 5 µm Cariact | Rh(NO₃)₃ | None | 8 | 49 |
| 18 | ^{b}5% Pd / Silica 5 µm Cariact | Pd(NO₃)₂ | None | 8 | 47 |
| ^{d}19 | ^{b}5% Pt / Silica 5 µm Cariact | Pt(NO₃)₂ | None | 8 | 69 |
| 20 | 0.5% Au 5% Rh / Silica 5 µm Cariact | Rh(NO₃)₃ | AuCl₃ | 9 | 48 |
| 21 | 0.5% Au 5% Rh / Silica 5 µm Cariact | Rh(NO₃)₃ | AuCl₃ | 14 | 50 |
| 22 | 2% Pd 2% Rh / Titania NorPro ST 61120 | Rh(NO₃)₃ | Pd(NO₃)₂ | 8 | 47 |
| 23 | 2% Pd 4% Pt / Titania NorPro ST 61120 | Pt(NO₃)₂ | Pd(NO₃)₂ | 7 | 38 |
| 24 | 4% Pt 2% Ru / Titania NorPro ST 61120 | ^{a}Ru(NO)(NO₃)ₓ(OH)_{y} | Pt(NO₃)₂ | 9 | 46 |
| 25 | 4% Pt 2% Rh / Titania NorPro ST 61120 | Rh(NO₃)₃ | Pt(NO₃)₂ | 8 | 60 |
| 26 | 4% Pt 2% Pd / Titania NorPro ST 61120 | Pd(NO₃)₂ | Pt(NO₃)₂ | 7 | 39 |
| 27 | 2% Ru 2% Pd / Titania NorPro ST61120 | Pd(NO₃)₂ | ^{a}Ru(NO)(NO₃)ₓ(OH)_{y} | 12 | 22 |
| 28 | 2% Ru 4% Pt / Titania NorPro ST 61120 | Pt(NO₃)₂ | ^{a}Ru(NO)(NO₃)ₓ(OH)_{y} | 8 | 38 |
| 29 | 2% Rh 2% Pd / Titania NorPro ST61120 | Pd(NO₃)₂ | Rh(NO₃)₃ | 7 | 46 |
| 30 | 2% Rh 4% Pt / Titania NorPro ST 61120 | Pt(NO₃)₂ | Rh(NO₃)₃ | 9 | 52 |
| 31 | ^{c}1.6% Rh 1.6% Pd / Silica Davisil 635 | Pd(NO₃)₂ | Rh(NO₃)₃ | 8 | 51 |
| 32 | ^{c}1.6% Rh 3.0% Pt / Silica Davisil 635 | Pt(NO₃)₂ | Rh(NO₃)₃ | 8 | 61 |
| 33 | ^{c}0.3% Rh 5.4% Pt / Silica Davisil 635 | Pt(NO₃)₂ | Rh(NO₃)₃ | 8 | 47 |
| 34 | ^{b}3.2% Pd / Silica Davisil 635 | Pd(NO₃)₂ | none | 8 | 46 |
| 35 | ^{b}6.0% Pt / Silica Davisil 635 | Pt(NO₃)₂ | none | 8 | 35 |
| 36 | ^{b}3.2% Rh / Silica Davisil 635 | Rh(NO₃)₃ | none | 8 | 30 |
| 37 | ^{c}0.6% Pt 2.9% Pt / Silica Davisil 635 | Pd(NO₃)₂ | Pt(NO₃)₂ | 8 | 45 |
| ^{e}38 | ^{c}1.65% Rh 4.7%Pt / Silica Davisil 635 | Pt(NO₃)₂ | Rh(NO₃)₃ | 8 | 89 |

| | | | | | |
|---|---|---|---|---|---|
| ^{a}Where x + y = 3 ^{b}Where no M2 was used, the M1/Support catalyst was calcined at 500 °C for 3 hours prior to use ^{c}Prepared by coimpregnation ^{d}This reaction was run for 6 hours ^{e}This reaction was conducted at 160 °C | | | | | |

## Claims

1. A process for preparing an adipic acid product, the process comprising:
reacting, in the presence of a hydrodeoxygenation catalyst and a halogen source, a hydrodeoxygenation substrate and hydrogen to convert at least a portion of the hydrodeoxygenation substrate to an adipic acid product,
wherein the adipic acid product is a product of formula II 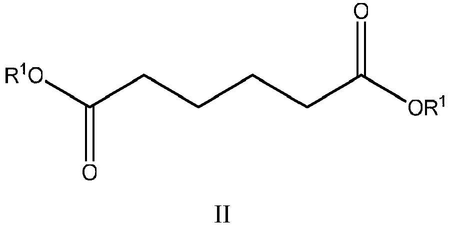 wherein the halogen source includes a hydrohalic acid or mixture thereof,
wherein the hydrodeoxygenation substrate comprises a compound of formula I 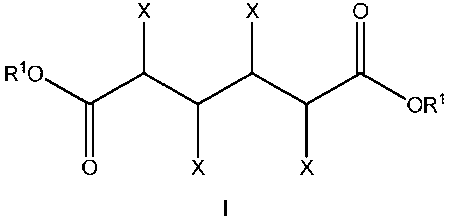 wherein X is independently hydroxyl, oxo, halo, acyloxy or hydrogen provided that at least one X is not hydrogen and R¹ is independently a salt-forming ion, hydrogen, or hydrocarbyl containing 1 to 18 carbon atoms, including branched or unbranched, and saturated or unsaturated species; or a mono- or di-lactone thereof.

2. The process as set forth in claim 1 wherein the hydrodeoxygenation substrate comprises a compound of formula I, wherein X is hydroxyl and R¹ is independently a salt-forming ion or hydrogen.

3. The process as set forth in claim 1 or 2 wherein the hydrodeoxygenation substrate comprises glucaric acid.

4. The process as set forth in any one of claims 1 to 3 wherein the adipic acid product comprises adipic acid.

5. The process as set forth in any one of claims 1 to 4 wherein at least a portion of the hydrodeoxygenation substrate is solubilized with a weak carboxylic acid.

6. The process as set forth in claim 5 wherein the weak carboxylic acid is acetic acid.

7. The process as set forth in any one of claims 1 to 6 wherein the hydrodeoxygenation catalyst comprises a heterogeneous catalyst.

8. The process as set forth in claim 7 wherein the hydrodeoxygenation catalyst comprises at least one d-block metal at a surface of a support selected from the group consisting of Ru, Rh, Pd, Pt, and combinations thereof.

9. The process as set forth in claim 8 wherein the hydrodeoxygenation catalyst comprises a first metal and a second metal, wherein the first metal is selected from the group consisting of Ru, Rh, Pd, Pt, and combinations thereof, and the second metal is selected from the group consisting of Mo, Ru, Rh, Pd, Ir, Pt, and Au, and wherein the second metal is not the same as the first metal.

10. The process as set forth in claim 8 or 9 wherein the hydrodeoxygenation catalyst support is selected from the group consisting of carbon, silica and zirconia.

11. The process as set forth in any one of claims 1 to 10 wherein the halogen source comprises hydrogen bromide.

12. The process as set forth in claim 1 wherein the halogen source comprises hydrogen iodide.

13. The process as set forth in any one of claims 1 to 12 wherein the molar ratio of halogen source to hydrodeoxygenation substrate is equal to or less than 1.

14. The process as set forth in any one of claims 1 to 13 wherein the reaction mixture is maintained at a temperature of at least 80°C.

15. The process as set forth in any one of claims 1 to 14 wherein the temperature of the reaction mixture is from 100°C to 200°C.

16. The process as set forth in any one of claims 1 to 15 wherein the reaction is conducted under a partial pressure of hydrogen ranging from 172 kPa (25 psia) to 17237 kPa (2500 psia).

17. The process as set forth in any one of claims 1 to 16 wherein at least a portion of the hydrodeoxygenation substrate is derived from glucose.

18. The process as set forth in any one of claims 1 to 17 wherein the process further comprises preparing the hydrodeoxygenation substrate by
reacting glucose with oxygen in the presence of an oxidation catalyst and in the absence of added base to convert at least a portion of the glucose to the hydrodeoxygenation substrate.

19. A process for preparing a downstream chemical product or intermediate, the process comprising:
preparing an adipic acid product as set forth in any one of claims 1 to 18; and
converting the adipic acid product to the downstream chemical product or intermediate wherein the downstream chemical product or intermediate is selected from the group consisting of adiponitrile, hexamethylene diamine, caprolactam, and 1,6-hexanediol.

20. A process for preparing a polymer, the process comprising:
preparing an adipic acid product as set forth in any one of claims 1 to 18; and
reacting the adipic acid product and a polymer precursor, thereby forming the polymer, wherein the polymer is selected from the group consisting of polyamide and polyester.

21. The process as set forth in claim 20 wherein the polyamide polymer comprises nylon 6,6.

22. A process for producing nylon 6, the process comprising:
preparing an adipic acid product as set forth in any one of claims 1 to 18; and
converting the adipic acid product to caprolactam, and
converting caprolactam into nylon 6.

23. A process for producing a salt of adipic acid, the process comprising:
preparing an adipic acid product as set forth in any one of claims 1 to 18; and
reacting adipic acid with hexamethylene diamine, thereby forming the salt.

24. The process as set forth in any one of claims 1 to 17 wherein the reaction is conducted at a temperature of at least 140 °C.

25. The process as set forth in claim 18 wherein the oxidation catalyst comprises metal selected from the group consisting of Pd, Pt, and combinations thereof on a catalyst support comprising a material selected from the group consisting of carbon, alumina, silica, titania, zirconia, and zeolite.

26. The process as set forth in claim 18 wherein the temperature of the reaction mixture is from 60°C to 150°C.

27. The process as set forth in claim 18 wherein the reaction is conducted under a partial pressure of oxygen in the range of from 104 kPa (15 psia) to 13790 kPa (2000 psia).

28. The process as set forth in claim 1 wherein the hydrodeoxygenation catalyst comprises a heterogeneous catalyst with at least one d-block metal selected from the group consisting of Co, Ni, Ru, Rh, Pd, Os, Ir, Pt, and combinations thereof at a surface of a support.

## Patentansprüche

1. Vorgang zum Herstellen eines Adipinsäureprodukts, wobei der Vorgang Folgendes umfasst:
Umsetzen eines Hydrodesoxygenierungssubstrats und Wasserstoffs in der Gegenwart eines Hydrodesoxygenierungskatalysators und einer Halogenquelle, um wenigstens einen Teil des Hydrodesoxygenierungssubstrats in ein Adipinsäureprodukt umzuwandeln,
wobei das Adipinsäureprodukt ein Produkt mit der Formel II ist: 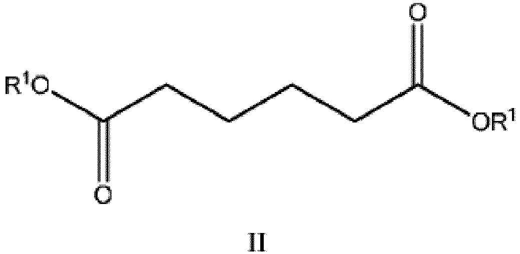 wobei die Halogenquelle eine hydrohalische Säure oder Mischung davon enthält, wobei das Hydrodesoxygenierungssubstrat eine Verbindung mit der Formel I umfasst: 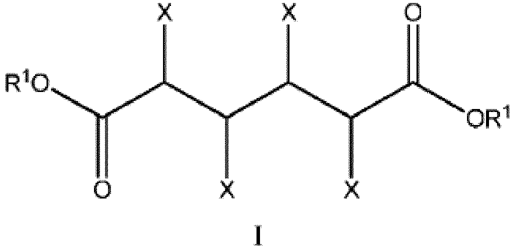 wobei X unabhängig Hydroxyl, Oxo, Halogen, Acyloxy oder Wasserstoff ist, vorausgesetzt, dass wenigstens ein X nicht Wasserstoff ist, und R₁ unabhängig ein salzbildendes Ion, Wasserstoff oder Hydrocarbyl mit 1 bis 18 Kohlenstoffatomen, einschließlich verzweigten oder unverzweigten und gesättigten oder ungesättigten Arten; oder ein Mono- oder Dilacton davon ist.

2. Vorgang nach Anspruch 1, wobei das Hydrodesoxygenierungssubstrat eine Verbindung mit der Formel I umfasst, wobei X Hydroxyl ist und R¹ unabhängig ein salzbildendes Ion oder Wasserstoff ist.

3. Vorgang nach Anspruch 1 oder 2, wobei das Hydrodesoxygenierungssubstrat Glucarsäure umfasst.

4. Vorgang nach einem der Ansprüche 1 bis 3, wobei das Adipinsäureprodukt Adipinsäure umfasst.

5. Vorgang nach einem der Ansprüche 1 bis 4, wobei wenigstens ein Teil des Hydrodesoxygenierungssubstrats mit einer schwachen Carbonsäure löslich gemacht wird.

6. Vorgang nach Anspruch 5, wobei die schwache Carbonsäure Essigsäure ist.

7. Vorgang nach einem der Ansprüche 1 bis 6, wobei der Hydrodesoxygenierungskatalysator einen heterogenen Katalysator umfasst.

8. Vorgang nach Anspruch 7, wobei der Hydrodesoxygenierungskatalysator wenigstens ein Metall des d-Blocks auf einer Oberfläche eines Trägers umfasst, das ausgewählt ist aus der Gruppe bestehend aus Ru, Rh, Pd, Pt und Kombinationen daraus.

9. Vorgang nach Anspruch 8, wobei der Hydrodesoxygenierungskatalysator ein erstes Metall und ein zweites Metall umfasst, wobei das erste Metall aus der Gruppe bestehend aus Ru, Rh, Pd, Pt und Kombinationen daraus ausgewählt ist und das zweite Metall aus der Gruppe bestehend aus Mo, Ru, Rh, Pd, Ir, Pt und Au ausgewählt ist und wobei das zweite Metall nicht das gleiche ist wie das erste Metall.

10. Vorgang nach Anspruch 8 oder 9, wobei der Hydrodesoxygenierungskatalysatorträger aus der Gruppe bestehend aus Kohlenstoff, Siliciumdioxid und Zirconiumdioxid ausgewählt ist.

11. Vorgang nach einem der Ansprüche 1 bis 10, wobei die Halogenquelle Bromwasserstoff umfasst.

12. Vorgang nach Anspruch 1, wobei die Halogenquelle lodwasserstoff umfasst.

13. Vorgang nach einem der Ansprüche 1 bis 12, wobei das Molverhältnis von Halogenquelle zu Hydrodesoxygenierungssubstrat höchstens 1 beträgt.

14. Vorgang nach einem der Ansprüche 1 bis 13, wobei das Reaktionsgemisch bei einer Temperatur von wenigstens 80 °C gehalten wird.

15. Vorgang nach einem der Ansprüche 1 bis 14, wobei die Temperatur des Reaktionsgemischs 100 °C bis 200 °C beträgt.

16. Vorgang nach einem der Ansprüche 1 bis 15, wobei die Reaktion unter einem Teildruck von Wasserstoff durchgeführt wird, der von 172 kPa (25 psia) bis 17.237 kPa (2.500 psia) reicht.

17. Vorgang nach einem der Ansprüche 1 bis 16, wobei wenigstens ein Teil des Hydrodesoxygenierungssubstrats von Glucose abgeleitet ist.

18. Vorgang nach einem der Ansprüche 1 bis 17, wobei der Vorgang ferner das Herstellen des Hydrodesoxygenierungssubstrats durch Folgendes umfasst:
Umsetzen von Glucose mit Sauerstoff in der Gegenwart eines Oxidationskatalysators und in der Abwesenheit von zugesetzter Base, um wenigstens einen Teil der Glucose in das Hydrodesoxygenierungssubstrat umzuwandeln.

19. Vorgang zum Herstellen eines nachgelagerten chemischen Produkts oder Zwischenprodukts, wobei der Vorgang Folgendes umfasst:
Herstellen eines Adipinsäureprodukts nach einem der Ansprüche 1 bis 18; und
Umwandeln des Adipinsäureprodukts in das nachgelagerte chemische Produkt oder Zwischenprodukt, wobei das nachgelagerte chemische Produkt oder Zwischenprodukt aus der Gruppe bestehend aus Adiponitril, Hexamethylendiamin, Caprolactam und 1,6-Hexandiol ausgewählt ist.

20. Vorgang zum Herstellen eines Polymers, wobei der Vorgang Folgendes umfasst:
Herstellen eines Adipinsäureprodukts nach einem der Ansprüche 1 bis 18; und
Umsetzen des Adipinsäureprodukts und eines Polymervorläufers, wodurch das Polymer ausgebildet wird, wobei das Polymer aus der Gruppe bestehend aus Polyamid und Polyester ausgewählt ist.

21. Vorgang nach Anspruch 20, wobei das Polyamidpolymer Nylon 6,6 umfasst.

22. Vorgang zum Herstellen von Nylon 6, wobei der Vorgang Folgendes umfasst:
Herstellen eines Adipinsäureprodukts nach einem der Ansprüche 1 bis 18; und
Umwandeln des Adipinsäureprodukts in Caprolactam und
Umwandeln von Caprolactam in Nylon 6.

23. Vorgang zum Herstellen eines Salzes von Adipinsäure, wobei der Vorgang Folgendes umfasst:
Herstellen eines Adipinsäureprodukts nach einem der Ansprüche 1 bis 18; und
Umsetzen von Adipinsäure mit Hexamethylendiamin, wodurch das Salz ausgebildet wird.

24. Vorgang nach einem der Ansprüche 1 bis 17, wobei die Reaktion bei einer Temperatur von wenigstens 140 °C durchgeführt wird.

25. Vorgang nach Anspruch 18, wobei der Oxidationskatalysator Metall, ausgewählt aus der Gruppe bestehend aus Pd, Pt und Kombinationen davon, auf einem Katalysatorträger umfasst, der ein Material, ausgewählt aus der Gruppe bestehend aus Kohlenstoff, Aluminiumoxid, Siliciumdioxid, Titandioxid, Zirconiumdioxid und Zeolith, umfasst.

26. Vorgang nach Anspruch 18, wobei die Temperatur des Reaktionsgemischs 60 °C bis 150 °C beträgt.

27. Vorgang nach Anspruch 18, wobei die Reaktion unter einem Teildruck von Sauerstoff im Bereich von 104 kPa (15 psia) bis 13.790 kPa (2.000 psia) durchgeführt wird.

28. Vorgang nach Anspruch 1, wobei der Hydrodesoxygenierungskatalysator einen heterogenen Katalysator mit wenigstens einem Metall des d-Blocks, ausgewählt aus der Gruppe bestehend aus Co, Ni, Ru, Rh, Pd, Os, Ir, Pt und Kombinationen davon, auf einer Oberfläche eines Trägers umfasst.

## Revendications

1. Processus de préparation d'un produit d'acide adipique, le processus comprenant :
la mise en réaction, en présence d'un catalyseur d'hydrodésoxygénation et d'une source d'halogène, d'un substrat d'hydrodésoxygénation avec un hydrogène pour convertir au moins une partie du substrat d'hydrodésoxygénation en un produit d'acide adipique,
le produit d'acide adipique étant un produit de la formule II 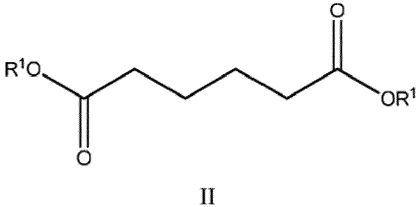 la source d'halogène comprenant un acide hydrohalique ou un mélange de celui-ci,
le substrat d'hydrodésoxygénation comprenant un composé de la formule I 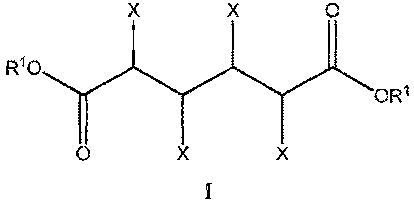 X représentant indépendamment un hydroxyle, un oxo, un halo, un acyloxy ou un hydrogène à condition qu'au moins un X ne représente pas un hydrogène et que R¹ représente indépendamment un ion formant des sels, un hydrogène ou un hydrocarbyle contenant de 1 à 18 atomes de carbone, y compris des espèces ramifiées ou non ramifiées et saturées ou non saturées ; ou un mono- ou di-lactone de ceux-ci.

2. Processus selon la revendication 1 dans lequel le substrat d'hydrodésoxygénation comprend un composé de la formule I, X représentant un hydroxyle et R¹ représentant indépendamment un ion formant des sels ou un hydrogène.

3. Processus selon la revendication 1 ou 2 dans lequel le substrat d'hydrodésoxygénation comprend de l'acide glucarique.

4. Processus selon l'une quelconque des revendications 1 à 3, le produit d'acide adipique comprenant de l'acide adipique.

5. Processus selon l'une quelconque des revendications 1 à 4 dans lequel au moins une partie du substrat d'hydrodésoxygénation est solubilisée avec un acide carboxylique faible.

6. Processus selon la revendication 5 dans lequel l'acide carboxylique faible est l'acide acétique.

7. Processus selon l'une quelconque des revendications 1 à 6 dans lequel le catalyseur d'hydrodésoxygénation comprend un catalyseur hétérogène.

8. Processus selon la revendication 7 dans lequel le catalyseur d'hydrodésoxygénation comprend au moins un métal de bloc d au niveau d'une surface d'un support choisi dans le groupe constitué de Ru, Rh, Pd, Pt et de leurs combinaisons.

9. Processus selon la revendication 8 dans lequel le catalyseur d'hydrodésoxygénation comprend un premier métal et un second métal, le premier métal étant choisi dans le groupe constitué de Ru, Rh, Pd, Pt et de leurs combinaisons, et le second métal étant choisi dans le groupe constitué de Mo, Ru, Rh, Pd, Ir, Pt et d'Au, et le second métal étant différent du premier métal.

10. Processus selon la revendication 8 ou 9 dans lequel le support de catalyseur d'hydrodésoxygénation est choisi dans le groupe constitué de carbone, de silice et de zircone.

11. Processus selon l'une quelconque des revendications 1 à 10 dans lequel la source d'halogène comprend du bromure d'hydrogène.

12. Processus selon la revendication 1 dans lequel la source d'halogène comprend de l'iodure d'hydrogène.

13. Processus selon l'une quelconque des revendications 1 à 12 dans lequel le rapport molaire entre la source d'halogène et le substrat d'hydrodésoxygénation est inférieur ou égal à 1.

14. Processus selon l'une quelconque des revendications 1 à 13 dans lequel le mélange réactionnel est maintenu à une température d'au moins 80°C.

15. Processus selon l'une quelconque des revendications 1 à 14 dans lequel la température du mélange réactionnel est comprise entre 100°C et 200°C.

16. Processus selon l'une quelconque des revendications 1 à 15 dans lequel la réaction est réalisée sous une pression partielle d'hydrogène comprise entre 172 kPa (25 psia) et 17 237 kPa (2 500 psia).

17. Processus selon l'une quelconque des revendications 1 à 16 dans lequel au moins une partie du substrat d'hydrodésoxygénation est dérivée du glucose.

18. Processus selon l'une quelconque des revendications 1 à 17, le processus comprenant en outre la préparation du substrat d'hydrodésoxygénation en
mettant en réaction du glucose avec de l'oxygène en présence d'un catalyseur d'oxydation et en l'absence d'une base ajoutée pour convertir au moins une partie du glucose en substrat d'hydrodésoxygénation.

19. Processus de préparation d'un produit chimique ou d'un intermédiaire en aval, le processus comprenant :
la préparation d'un produit d'acide adipique selon l'une quelconque des revendications 1 à 18 ; et
la conversion du produit d'acide adipique en un produit chimique ou intermédiaire en aval, le produit chimique ou intermédiaire en aval étant choisi dans le groupe constitué d'adiponitrile, d'hexaméthylènediamine, de caprolactame et de 1,6-hexanediol.

20. Processus de préparation d'un polymère, le processus comprenant :
la préparation d'un produit d'acide adipique selon l'une quelconque des revendications 1 à 18 ; et
la mise en réaction du produit d'acide adipique et d'un précurseur de polymère, formant ainsi le polymère, le polymère étant choisi dans le groupe constitué de polyamide et de polyester.

21. Processus selon la revendication 20 dans lequel le polymère de polyamide comprend du nylon 6,6.

22. Processus de production d'un nylon 6, le processus comprenant :
la préparation d'un produit d'acide adipique selon l'une quelconque des revendications 1 à 18 ; et
la conversion du produit d'acide adipique en caprolactame, et
la conversion de caprolactame en nylon 6.

23. Processus de production d'un sel d'acide adipique, le processus comprenant :
la préparation d'un produit d'acide adipique selon l'une quelconque des revendications 1 à 18 ; et
la mise en réaction d'acide adipique avec l'hexaméthylènediamine, formant ainsi le sel.

24. Processus selon l'une quelconque des revendications 1 à 17 dans lequel la réaction est réalisée à une température d'au moins 140°C.

25. Processus selon la revendication 18 dans lequel le catalyseur d'oxydation comprend un métal choisi dans le groupe constitué de Pd, de Pt et de leurs combinaisons sur un support de catalyseur comprenant une matière choisie dans le groupe constitué de carbone, d'alumine, de silice, de titane, de zircone et de zéolite.

26. Processus selon la revendication 18 dans lequel la température du mélange réactionnel est comprise entre 60°C et 150°C.

27. Processus selon la revendication 18 dans lequel la réaction est réalisée sous une pression partielle d'oxygène dans la plage comprise entre 104 kPa (15 psia) et 13 790 kPa (2 000 psia).

28. Processus selon la revendication 1 dans lequel le catalyseur d'hydrodésoxygénation comprend un catalyseur hétérogène avec au moins un métal de bloc d choisi dans le groupe constitué de Co, Ni, Ru, Rh, Pd, Os, Ir, Pt et de leurs combinaisons au niveau d'une surface d'un support.
